# EUROPEAN PATENT SPECIFICATION

(11) **EP 3 773 456 B1**
(45) Date of publication and mention of the grant of the patent: **28.08.2024**
(21) Application number: 19721327.5
(22) Date of filing: 08.05.2019
(51) Int. Cl.: A61K 8/44, A61K 8/46, A61Q 5/00, A61Q 5/02, A61K 8/73, A61K 8/20

(54) **CLEANSING COMPOSITION**
REINIGUNGSMITTELZUSAMMENSETZUNG
COMPOSITION DE NETTOYAGE

(30) Priority: 11.05.2018 EP 18171887
(43) Date of publication of application: 17.02.2021
(73) Proprietor: Unilever IP Holdings B.V., 3013 AL Rotterdam (NL); Unilever Global IP Limited, Wirral, Merseyside CH62 4ZD (GB)
(72) Inventor: GALPIN, Annie, Jaye, Wirral Merseyside CH63 3JW (GB); MACHEN, Robert, Wirral Merseyside CH63 3JW (GB); PUNTAMBEKAR, Smita, Wirral Merseyside CH63 3JW (GB); RILEY, Robert, George, Wirral Merseyside CH63 3JW (GB); STARCK, Pierre, Wirral Merseyside CH63 3JW (GB)
(74) Representative: Chisem, Janet
(86) International application number: PCT/EP2019/061865
(87) International publication number: WO 2019/215254

(56) References cited:
- FR-A1- 3 021 531
- US-A- 5 811 087
- US-A- 5 840 670
- US-A1- 2017 319 453
- US-A1- 2017 340 540
- US-A1- 2017 360 688
- US-A1- 2018 071 198
- US-B1- 6 258 763
- "Disinfectant Concentrates as Additives in Various Compositions and Uses", IP.COM JOURNAL, IP.COM INC., WEST HENRIETTA, NY, US, 20 March 2008 (2008-03-20), XP013124304, ISSN: 1533-0001

## Description

### Field of the Invention

The subject invention relates to mild sulphate free cleansing compositions for hair and skin, including scalp.

### Background and Prior Art

Microstructure and rheology are important factors in personal care formulations, as they affect consumer acceptance as well as product performance. The selection, amount and relative amount of surfactant contributes to the microstructure of a personal cleansing composition. In turn, microstructure can impact rheological properties such as composition viscosity and viscosity building characteristics, and may also contribute to composition stability.

A sufficient level of surfactant is ordinarily needed for surfactant molecules to be able to assemble into micelles, and for the micelles to aggregate to build structure. Commercially available water-based cleansing compositions frequently contain upwards of 12 weight percent of surfactant. The major surfactant component of such compositions is commonly an alkyl and/or alkyl ether sulfate surfactant, with lauryl and laureth sulfates, surfactants known to afford good detergency, being among the sulfate surfactants commonly employed. Sulfate surfactants belong to a class of materials known as anionic surfactants. The sulfate surfactants are frequently used together with an amphoteric co-surfactant, with betaine surfactants such as cocamidopropyl betaine being among the amphoteric surfactants commonly employed. Betaine surfactants help to boast lather and are generally milder than sulfate surfactants, albeit without the detergent power of the sulfate surfactants. Advantageously, personal cleansing compositions based on sulfate surfactant can normally be thickened by the addition of simple salts.

Despite the widespread use of sulfate surfactants in personal cleansing compositions, there is considerable interest in milder alternatives, including compositions in which the anionic surfactant is a sulfate free surfactant.

Achieving an acceptable composition viscosity is an important factor in providing a mild personal cleansing composition that can be applied in a controlled manner and readily spread in use. Composition viscosity, together with attributes such as foamability can also impact consumer perception of such products. When sulfate surfactants are eliminated, developing a microstructure that results in desirable rheological properties can be challenging, particularly in the case of mild compositions with low surfactant concentrations; additionally, building the viscosity of such compositions by the addition of a simple salt can be problematic. Eliminating sulfate surfactants can also be problematic in regard to formulating mild cleansing compositions that are stable at acidic pH.

One approach to the issue of thickening systems that are free of sulfate surfactants has been to use sulfate-free surfactants together with polymeric thickeners. Polymeric thickeners can have a gelation effect that transforms a product with what is ordinarily Newtonian rheology under conditions of low shear, such as is experienced, for example, during dosing, application and spreading in-use, to a non-Newtonian rheology. In addition to being detectable by a consumer as an undesirable departure from product norms, the use of such thickeners can further limit the ability to subsequently adjust composition viscosity through the use of simple salts.

Employing relatively high levels of non-sulfate surfactant may also assist in building the viscosity of systems that are free of sulfate surfactants. The levels of non-sulfate surfactant needed may, however, be higher than conventional norms, and may also result in non-isotropic surfactant systems having relatively non-labile liquid crystalline structures or domains. In contrast to isotropic surfactant systems, which tends to promote foaming efficiency, the relatively labile microstructure of liquid crystalline microstructures tend to "trap" surfactant and impair foamability. Additionally, liquid crystalline structures or domains may impede light transmission and may impart a turbid or cloudy appearance to a composition, which may be problematic where translucency is desired.

There is a need for mild sulphate free cleansing compositions, suitable for hair, skin and scalp, having desirable rheological properties, including compositions with relatively low surfactant concentrations. Of particular interest are low surfactant content compositions free of sulfate surfactants, the viscosity of which compositions may be increased through the addition of electrolytes such as simple salts.

WO18002557A1 uses high concentrations (35-60%) of surfactant mixtures including sulfate free anionic, amphoteric and non-ionic that access the lamellar phase region to control the rheology. These formulas have no added salt. Although high surfactant concentrations in lamellar phase solve the rheology build problem, they may not favour mild benefits or have the in-use application and sensory characteristics of an isotropic shampoo, such as transparent appearance and flash foam.

US2017319453A teaches that a combination of Alpha Olefin Sulfonate (AOS) plus a glycerin fatty acid ester and an amphoteric surfactant (a betaine) give no irritation or reduced irritation, and superior foam quality, and may be stable, in particular stable over time and/or under elevated temperature. Under acidic pH, ester bonds are prone to attack and can become unstable, which tends to thin an isotropic formula over time. Heat will accelerate these destabilising reactions.

US2016095804A uses complex combinations of sulfate free surfactants (e.g. anionic surfactant plus amphoteric surfactants with optional non-ionic surfactants) but structures them with hydrophobically modified, high molecular weight polymers. It also employs cationic conditioning agents (mixtures of polymers and silicones or functional silicones). However, we have found that the addition of structuring polymers to a cleansing formula can result in adverse sensory performance in the form of poor clean feel, coating and stickiness.

US2017079899A & US2017095410A reveal that blends of Alpha Olefin Sulfonate with different chain lengths (C16 + C18) at high concentrations and at specific ratios require a suspending agent in the form of a polymeric material to thicken the formula. The use of amphoteric or zwitterionic materials such as betaines and the inclusion of at least one foam booster such as a fatty material are also envisaged.

US2012157365A uses a mixture of polyglyceryl non-ionic, amphoteric and sulfate free anionic surfactants at pH less than 5.4 to employ organic acids such as sodium benzoate as the preservative. Ratios of polyglyceryl to amphoteric range from 0.05:1 to 3:1 and sulfate free anionic to amphoteric ranges from 0.3:1 to 4:1.

US5811087 discloses aqueous hair shampoos containing a combination of
a) 1% to 25% wt. of at least one alkyl amidoether carboxylic acid of a defined formula; b) 1% to 25% wt. of at least one anionic sulfate or sulfonate surfactant; c) 0.1% to 10% wt. of at least one compound selected from C8-C18-acylmono- and-dialkanolamides, surface-active betaines and sulfobetaines and (or) surface-active amine oxides; and
d) 0.05% to 5% by wt. of at least one cationic polymer, having a charge density of at least 2.50 meq/g. An example includes alpha olefin sulphonate and lauryl hydroxy sultaine.

US2017/0340540 discloses cosmetic compositions, for cleansing and caring for keratinous substrates, comprising: (i) one or more linear tr.-olefin sulfonates, (ii) one or more non-oxyalkylenated anionic surfactants other than the compounds (i), present at 1% to 20% by weight; and (iii) one or more additional surfactants chosen from amphoteric surfactants and nonionic surfactants. An example includes alpha olefin sulphonate and cocobetaine along with 0.7 wt % of polyquaternium-10.

US2017/0360688 reveals the use of a combination of anionic and cationic polyelectrolytes in cosmetic compositions also containing at least one surfactant. Stable viscosity and yield stress are achieved.

The inventors have identified Alpha Olefin Sulfonate (AOS) as a sulfate free, primary surfactant. As with sulfate based chassis a secondary surfactant is required to help build viscosity using salt, control the foam and aid in the delivery of mildness benefits through lower CMC.

It is desirable to reduce the level of surfactant used in formulations (for mildness and environmental benefits). We have found, however, that if you reduce the amount of surfactant, then the viscosity of the formulation falls undesirably.

In addition, the typical secondary surfactant, cocamidopropyl betaine (CAPB) does not allow viscosity to be built at low total surfactant concentrations with Alpha Olefin Sulfonate (AOS) as the primary surfactant.

The inventors have now found that a combination, of AOS, at a defined ratio, with at least one of an alkyl betaine, an alkyl hydroxy sultaine, an alkyl aminopropyl hydroxy sultaine or an alkyl amphoacetate can afford viscosity builds with salt addition at low total concentrations of surfactant. This negates the need for other thickening agents, for example, polymers and other secondary surfactants.

Compositions in accordance with the invention having a combination of anionic and amphoteric sulfate free surfactants at enriched amphoteric ratios; reduced surfactant concentrations; specific primary sulfate free surfactant levels and a cationic polymer, gives good foamability, excellent cleaning, wet detangling and desirable rheological characteristics, whilst maintaining mildness to skin and hair protein.

Compositions having a transparent appearance can be produced if no silicone and no further thickening agents are added.

### Definition of the Invention

In a first aspect the present invention provides a sulphate free cleansing composition for hair,scalp and skin comprising, in an aqueous continuous phase:
a total amount of anionic surfactant, amphoteric surfactant and zwitterionic surfactant consisting of:
   (i) from 3 wt % to 13 wt %, by weight of the total composition at 100 % activity, of an alpha olefin sulfonate anionic surfactant of general formula (I):

      R¹-CH=CH-CH₂-SO₃⁻M⁺ (I)

      in which R¹ is selected from linear or branched alkyl groups having from 11 to 13 carbon atoms and mixtures thereof; and M is a solubilizing cation;
   (ii) from 1 to 6%, by weight of an amphoteric or zwitterionic surfactant, selected from an alkyl betaine of general formula (II)

      R²-N⁺(CH₃)₂-CH₂-COO⁻M⁺ (II)

      wherein R² = C12 (Lauryl) or Coco derived;
      an alkyl hydroxy sultaine of general formula (III),

         R³-N⁺(CH₃)₂-CH₂-CH(OH)-CH₂-SO₃⁻M⁺ (III)
      wherein R³ = C12 (Lauryl) or Coco derived;
      an alkyl aminopropyl hydroxy sultaine of general formula (IV),

         R⁴-CO-NH-(CH₂)₃-N⁺(CH₃)₂-CH₂-CH(OH)-CH₂-SO₃⁻M⁺ (IV)
      wherein R⁴ = C12 (Lauryl) or Coco derived;
      an alkyl amphoacetate of general formula (V),

         R⁵-CO-NH-(CH₂)₂-N(CH₂-CH₂-OH)(CH₂-COO⁻ M⁺) (V)
      wherein R⁵ = C12 (Lauryl) or Coco derived;
      and mixtures thereof; wherein no further anionic, amphoteric and zwitterionic surfactants are present;
   (iii) from 0.05 wt % to 0.5 wt % of a cationic polymer;
   (iv) an inorganic electrolyte; and
   (v) water;
in which the weight ratio of (i) to (ii) ranges from 1:1 to 6:1 and the pH of the composition is from 3 to 6.5;
   wherein the composition is free from thickening agents selected from thickening polymers and secondary surfactants not defined in (ii); and
   wherein no other surfactants are present.

In a second aspect, the invention provides a method of treating hair and/or scalp and/or skin comprising the step of applying to the hair and/or scalp and/or skin a composition as defined by the first aspect.

Preferably the method comprises an additional step of massaging the composition of the first invention into the hair and/or scalp and/or skin.

Preferably the method comprises an additional step of rinsing the hair and/or scalp and/or skin.

### Detailed Description of the Invention

All molecular weights as used herein are weight average molecular weights, unless otherwise specified.

### Aqueous continuous phase

By "aqueous continuous phase" is meant a continuous phase which has water as its basis.

Suitably, the composition of the invention will comprise from about 75 to about 95%, preferably from 85 to 95%, more preferably from 87 to 95% water (by weight based on the total weight of the composition).

Preferably the composition comprises an isotropic surfactant phase, where under dilution, isotropic micelles provide higher availability of monomers to the air/water interface, whereas anisotropic may diffuse at a slower rate, resulting in lower flash foam properties. Thus, the isotropic phase is advantageous for product appearance, clarity and good flash foam properties.

All amounts referred to herein are based on 100 % activity (or "active") unless otherwise stated. By 100 % activity (or "active") is meant that the material is not diluted and is at 100 % v/v or wt/wt. Many materials used in personal care formulations are commercially available at different active concentrations, for example at 70 % active or 60 % active. For example, 100 ml of 70 % active surfactant provides the same amount of active material as 70 ml of 100 % active surfactant. Therefore, in order to provide for variations in activities of materials, all amounts are based on 100 % active materials.

The aqueous continuous phase comprises a total amount of anionic, amphoteric and zwitterionic surfactant consisting of (i) and (ii) below. That is to say, no further anionic, amphoteric and zwitterionic surfactants are present in the compositions of the invention. No other surfactants, for example, nonionic surfactants are present in the compositions of the invention.

### (i) The alpha olefin sulfonate anionic surfactant

The composition of the invention comprises (i) one or more alpha olefin sulfonate anionic surfactants of general formula (I)

R¹-CH=CH-CH₂-SO₃⁻M⁺ (I)

in which R¹ is selected from linear or branched alkyl groups having from 11 to 13 carbon atoms and mixtures thereof; and M is a solubilizing cation;

Preferably R¹ in general formula (I) is a C₁₄ or C₁₆ linear alkyl group.

Preferably M in general formula (I) is selected from alkali metal cations (such as sodium or potassium), ammonium cations and substituted ammonium cations (such as alkylammonium, alkanolammonium or glucammonium).

Commercially produced alpha olefin sulfonate anionic surfactants of general formula (I) may be made by sulfating C14-16 olefins derived from natural gas. The process can also yield mixtures of homologues and low levels of unreacted olefins.

Particularly preferred is alpha olefin sulfonate with an average of 14-16 carbons. A suitable example of such a material is Bioterge AS40 (ex Stepan).

The amount of alpha olefin sulfonate anionic surfactant, at 100 % activity, of general formula (I) ranges from 3 to 13 %, for example from 3 to 12.85 %, preferably from 3.5 to 12%, more preferably from 3 to 10%, still more preferably from 3 to 9% and most preferably from 3.25 to 8% (by weight based on the total weight of the composition).

### (ii) The amophoteric or zwitterionic surfactant of general formulae (II), (III), (IV) or (V)

The composition of the invention comprises (ii) an amphoteric or zwitterionic surfactant, selected from an alkyl betaine of general formula (II)

R²-N⁺(CH₃)₂-CH₂-COO⁻M⁺ (II)

wherein R² = C12 (Lauryl) or Coco derived;
an alkyl hydroxy sultaine of general formula (III),

   R³-N⁺(CH₃)₂-CH₂-CH(OH)-CH₂-SO₃⁻M⁺ (III)
wherein R³ = C12 (Lauryl) or Coco derived;
an alkyl aminopropyl hydroxy sultaine of general formula (IV),

   R⁴-CO-NH-(CH₂)₃-N⁺(CH₃)₂-CH₂-CH(OH)-CH₂-SO₃⁻M⁺ (IV)
wherein R⁴ = C12 (Lauryl) or Coco derived;
   an alkyl amphoacetate of general formula (V),

   R⁵-CO-NH-(CH₂)₂-N(CH₂-CH₂-OH)(CH₂-COO⁻ M⁺) (V)
wherein R⁵ = C12 (Lauryl) or Coco derived;
and mixtures thereof.

The preferred surfactant (ii) is selected from coco betaine, lauryl hydroxy sultaine, coco aminopropyl hydroxy sultaine, lauryl amphoacetate and mixtures thereof, most preferably selected from coco betaine, lauryl hydroxy sultaine and mixtures thereof.

The amount of amphoteric or zwitterionic surfactants of general formula (II), (III), (IV) or (V) or mixtures thereof, preferably ranges from 1 to 6%, more preferably from 1 to 5%, most preferably from 1.2 to 4 % (based on the total weight of the composition and 100 % activity).

In a preferred composition according to the invention the amphoteric or zwitterionic surfactant (ii) is selected from coco betaine, lauryl hydroxy sultaine, coco aminopropyl hydroxy sultaine, lauryl amphoacetate and mixtures thereof, in an amount ranging from 1 to 4 % (by weight based on the total weight of the composition and 100 % activity).

In a more preferred composition the amphoteric or zwitterionic surfactant (ii) is selected from a betaine amphoteric surfactant of general formula (II), which is coco betaine, an amphoteric surfactant of general formula (III), which is lauryl hydroxy sultaine, and mixtures thereof, in an amount of from 1 to 4 % (by weight based on the total weight of the composition and 100 % activity).

An especially preferred composition according to the invention comprises (i) alpha olefin sulfonate in an amount ranging from 3.25 to 8% (by weight based on the total weight of the composition and 100 % active material); and (ii) an amphoteric or zwitterionic surfactant selected from coco betaine, lauryl hydroxy sultaine, coco aminopropyl hydroxy sultaine, lauryl amphoacetate or mixtures thereof, in an amount ranging from 1 to 4 % (by weight based on the total weight of the composition and 100 % active material).

The combined amount of (i) and (ii) ranges from 4 to 19 wt %, preferably from 5 to 15 wt %, most preferably from 5 to 11 wt % (based on the total weight of the composition and 100 % activity).

The weight ratio of the alpha olefin sulfonate anionic surfactant (i) to the amphoteric surfactant (ii) ranges from 1:1 to 6:1, preferably from 1.5:1 to 4.5:1 and most preferably 2:1 to 4:1.

The pH of the composition of the invention ranges from 3 to 6.5, preferably from 3.5 to 5.1, more preferably from 4 to 5.

A protonating agent may be used for achieving the low pH. Suitable protonating agents are acids. Suitable acids useful herein include hydrochloric acid, citric acid, acetic acid, tartaric acid, fumaric acid, lactic acid, malic acid, succinic acid, and mixtures thereof. Preferably, the acid is selected from the group consisting of citric acid, acetic acid, tartaric acid, hydrochloric acid, fumaric acid, lactic acid and mixtures thereof.

### (iii) The cationic polymer

The composition of the invention comprises (iii) one or more cationic polymers. Such polymers may enhance the delivery of wet feel benefits in the composition.

Cationic polymers for use in the invention suitably have a cationic charge density ranging from about 0.3 to about 4 meq/g, preferably from about 0.4 to about 3.5 meq/g. The term "cationic charge density" in the context of this invention refers to the ratio of the number of positive charges on a monomeric unit of which a polymer is comprised to the molecular weight of the monomeric unit. The charge density multiplied by the polymer molecular weight determines the number of positively charged sites on a given polymer chain. Cationic charge density can be determined according to the Kjeldahl Method. Those skilled in the art will recognize that the charge density of amino-containing polymers may vary depending upon pH and the isoelectric point of the amino groups. The charge density should be within the above limits at the pH of intended use.

Suitable cationic polymers for use in the invention include cationic polysaccharide derivatives, such as cationic cellulose derivatives, cationic starch derivatives, and cationic guar gum derivatives.

Preferred cationic polysaccharide derivatives for use in the invention include cationic guar gum derivatives and cationic cellulose derivatives.

Examples of preferred cationic guar gum derivatives for use in the invention include guar hydroxypropyltrimethylammonium chlorides. Guar hydroxypropyltrimethylammonium chlorides for use in the invention are generally comprised of a nonionic guar gum backbone that is functionalized with ether-linked 2-hydroxypropyltrimethylammonium chloride groups, and are typically prepared by the reaction of guar gum with N-(3-chloro-2-hydroxypropyl) trimethylammonium chloride.

Guar hydroxypropyltrimethylammonium chlorides for use in the invention generally have an average molecular weight (weight average molecular mass (M_{w}) determined by size exclusion chromatography) in the range 500,000 to 3 million g/mol, more preferably 800,000 to 2.5 million g/mol.

Guar hydroxypropyltrimethylammonium chlorides for use in the invention (preferably guar hydroxypropyltrimethylammonium chlorides) generally have a charge density ranging from 0.5 to 1.8 meq/g.

Examples of preferred cationic cellulose derivatives for use in the invention include poly(1,2-oxyethanediyl)-2-hydroxy-3-trimethylammonium propyl chloride cellulose ethers (INCI: Polyquaternium-10).

Preferably, the cationic polymer is selected from Polyquaternium 10, guar hydroxypropyltrimethylammonium chlorides having a M_{w} ranging from 800,000 to 2.5 million g/mol and a charge density ranging from 0.5 to 1.8 meq/g, and mixtures thereof.

Mixtures of any of the above described cationic polymers may also be used.

In a typical composition according to the invention the amount of cationic polymer will generally range from 0.05 wt % to 0.5 wt % and preferably ranges from 0.15 wt % to 0.5 wt % based on the total weight of the composition.

In a preferred composition according to the invention the one or more cationic polymers are selected from guar hydroxypropyltrimethylammonium chlorides having a M_{w} ranging from 800,000 to 2.5 million g/mol and a charge density ranging from 0.5 to 1.8 meq/g; in an amount ranging from 0.15 to 0.3% (by weight based on the total weight of the composition).

Another class of suitable cationic conditioning polymers are the high molecular weight Polyethylene Glycol (PEG) polymers, for example PEG 45M, available as Polyox from Dow.

Mixtures of any of the above described polymers may also be used.

### The inorganic electrolyte

We have found that, surprisingly, the compositions of the invention are amenable to building viscosity very well. It is thus possible to build viscosity at lower concentrations at enriched surfactant ratios. This is further advantage of the invention.

The composition of the invention includes at least one inorganic electrolyte. The inorganic electrolyte provides viscosity to the composition.

The viscosity of the composition ranges from 3,500 to 15,000 mPa.s, most preferably from 4,000 to 12,000 mPa.s when measured using a Brookfield V2 viscometer (spindle RTV5, 1 minute, 20rpm) at 30°C.

At these range our products are pourable yet thick enough to satisfy the consumer desire for thick compositions.

Suitable inorganic electrolytes include metal chlorides (such as sodium chloride, potassium chloride, calcium chloride, magnesium chloride, zinc chloride, ferric chloride and aluminium chloride) and metal sulfates (such as sodium sulfate and magnesium sulfate).

It is intended that the inorganic electrolyte is separate from any inorganic electrolytes that may be present in the raw materials of the invention.

Examples of preferred inorganic electrolytes for use in the invention include sodium chloride, potassium chloride, magnesium sulfate and mixtures thereof.

Mixtures of any of the above described materials may also be suitable.

The amount of inorganic electrolyte in compositions of the invention preferably ranges from 0.5 to 10 %, more preferably from 0.75 to 7 %, even more preferably from 1 to 5 % and most preferably from 1 to 3 % (by weight based on the total weight of the composition).

A preferred composition of the invention has a weight ratio of (i) an alpha olefin sulfonate anionic surfactant of general formula (I) to (ii) an amphoteric or zwitterionic surfactant of general formula (II), (III), (IV), (V) or mixtures thereof, of from 2:1 to 4:1 and comprises an amount of inorganic electrolyte of from 1 to 3 wt % based on total weight of the composition.

A further preferred composition of the invention has a weight ratio of (i) an alpha olefin sulfonate anionic surfactant of general formula (I) to (ii) an amphoteric or zwitterionic surfactant of general formula (II), (III), (IV), (V) or mixtures thereof, of from greater than 4:1 to 6:1, preferably 5:1 to 6:1 and comprises an amount of inorganic electrolyte of from 1 to 5, preferably from greater than 3 to 5 wt %, more preferably 4 to 5 wt % based on total weight of the composition.

Preferably, the compositions of the invention are free from silicone. In the context of the invention, by free from is meant having less than 0.4 weight %, more preferably less than 0.1 weight %, even more preferably less than 0.05 weight %, still more preferably less than 0.001 weight %, yet preferably less than 0.0001 weight %, and most preferably 0 weight % of silicone by weight of the total composition.

The compositions of the invention are free from thickening agents selected from thickening polymers and secondary surfactants not included in (ii). In the context of the invention, by free from is meant having less than 0.4 weight %, more preferably less than 0.1 weight %, even more preferably less than 0.05 weight %, still more preferably less than 0.001 weight %, yet preferably less than 0.0001 weight %, and most preferably 0 weight % of thickening agents by weight of the total composition. For the sake of clarity, the cationic polymer (iii) of the invention is not intended to be a thickening polymer.

Most preferably, the compositions of the inventions are free from silicones. They are free from thickening agents as defined above.

Preferably, the composition is transparent. Where the composition is transparent, it is free from materials that cause cloudiness, such as silicones, structurants (for example carbomer) and visco-surfactants (for example cocoamide monoethanolamide (CMEA)).

In the context of the invention, by free from materials that cause cloudiness is meant having less than 0.4 weight %, more preferably less than 0.1 weight %, even more preferably less than 0.05 weight %, still more preferably less than 0.001 weight %, yet more preferably less than 0.0001 weight % and most preferably 0 weight %, of materials that cause cloudiness, by weight of the total composition, such that a transparent composition in obtained.

### A preservative

The composition of the invention preferably comprises one or more preservatives, selected from sodium benzoate, sodium salicylate, benzyl alcohol, phenoxyethanol, 1,2-alkanediols, lodopropynyl butylcarbamate (IPBC), 5-chloro-2-methyl-2H-isothiazol-3-one, 2-methyl-2H-isothiazol-3-one, or mixtures thereof. Preferably the preservative is an organic acid, most preferably the preservative is sodium benzoate.

A preferred composition has a pH of from 3 to 5, preferably 4 to 5 and comprises a preservative that is sodium benzoate.

### Optional Ingredients

Preferably, the composition of the invention further comprises one or more structurants to assist in the suspension of dispersed benefit agent and provide phase stability. Suitable structurants include polyacrylic acids, polymethacrylic acids, cross-linked polymers of acrylic acid, cross-linked polymers of methacrylic acid, copolymers of acrylic acid with a hydrophobic monomer, copolymers of methacrylic acid with a hydrophobic monomer, copolymers of carboxylic acid-containing monomers and acrylic esters, copolymers of carboxylic acid-containing monomers and methacrylic esters, cross-linked copolymers of acrylic acid and acrylate esters, cross-linked copolymers of methacrylic acid and acrylate esters heteropolysaccharide gums and crystalline long chain acyl derivatives.

Preferred structurants are selected from polyacrylic acids, polymethacrylic acids, cross-linked polymers of acrylic acid, cross-linked polymers of methacrylic acid and mixtures thereof.

Mixtures of any of the above structurants may be used.

When included, the total amount of structurant is generally 0.1 to 10 %, preferably from 0.1 to 3 %, more preferably from 0.2 to 2 %, most preferably from 0.3 to 0.9 % (by weight based on the total weight of the composition).

A preferred composition comprises a structurant selected from polyacrylic acids, polymethacrylic acids, cross-linked polymers of acrylic acid, cross-linked polymers of methacrylic acid and mixtures thereof in an amount of from 0.1 to 10 %, preferably from 0.1 to 3 %, more preferably from 0.2 to 2 %, most preferably from 0.3 to 0.9 % (by weight based on the total weight of the composition).

A composition of the invention may contain further optional ingredients to enhance performance and/or consumer acceptability. Examples of such ingredients include fragrance, dyes and pigments. Each of these ingredients will be present in an amount effective to accomplish its purpose. Generally, these optional ingredients are included individually at an amount of up to 5% (by weight based on the total weight of the composition).

The composition of the invention is primarily intended for topical application to the hair, scalp or skin.

Most preferably the composition of the invention is topically applied to the hair, scalp or skin and then massaged into the hair, scalp or skin. The composition is then rinsed off the hair, scalp or skin with water prior to drying the hair, scalp or skin.

The invention will be further illustrated by the following Examples.

### EXAMPLES

### Example 1: Preparation of Compositions 1 to 9 in accordance with the invention and Comparative Composition A

Rinse-off aqueous hair cleansing shampoo formulations were prepared, having ingredients as shown in Table 1 below.

All the shampoos were prepared using the following method:
1. A vessel was charged with water. Surfactants and any structurant were added with stirring.
2. The mixture was heated to 30° C and mixed until completely homogenous.
3. Any cationic polymer was then added and mixed well.
4. Any preservative was added.
5. The pH was adjusted to pH 4.5 using citric acid.
6. Salt was then added to adjust the viscosity.

**Table 1: Compositions 1 to 9 in accordance with the invention and Comparative Composition A.**

| **INCI and/or Trade Name** | **% active** | **A** | **1** | **2** | **3** | **4** | **5** | **6** | **7** | **8** | **9** |
|---|---|---|---|---|---|---|---|---|---|---|---|
| Alpha Olefin Sulfonate (Bioterge AS-40) | 38.5 | 20.7 8 | 19.0 9 | 19.0 9 | 24.2 4 | 13.8 5 | 19.0 4 | 16.6 2 | 17.8 2 | 24.4 9 | 13.8 4 |
| Cocamidopropyl betaine / Tego betain CK KB5 | 30 | 13.3 3 | | | | | | | | | |
| Coco betaine / Genagen KB | 30 | | 8.33 | | | | 12.2 2 | 5.33 | 3.8 | 5.23 | 8.9 |
| Lauryl Hydroxy Sultaine / Mackam LHS E | 50 | | | 5.3 | | | | | | | |
| Cocamidopropyl Hydroxy Sultaine / Mackam 50-SB | 42 | | | | | 6.35 | | | | | |
| Sodium lauroamphoaceta te / Miranol Ultra L32 | 32 | | | | 14.5 8 | | | | | | |
| Polyquaternium 10 | 100 | | 0.05 | 0.05 | 0.05 | 0.05 | 0.2 | 0.2 | 0.2 | | |
| Guar Hydroxypropyltrimonium Chloride | 100 | | | | | | | | | 0.2 | 0.2 |
| Sodium Benzoate | 100 | 0.5 | | | 0.5 | 0.5 | 0.5 | 0.5 | 0.5 | 0.5 | 0.5 |
| Disodium EDTA | 100 | 0.05 | | | 0.05 | 0.05 | 0.05 | 0.05 | 0.05 | 0.05 | 0.05 |
| Citric acid | 100 | to pH 4.5 | to pH 4.5 | to pH 4.5 | to pH 4.5 | to pH 4.5 | to pH 4.5 | to pH 4.5 | to pH 4.5 | to pH 4.5 | to pH 4.5 |
| Sodium Chloride | 100 | 3 | 2.26 | 3.25 | 3 | 3 | 1 | 3 | 5 | 4 | 1 |
| Water | 100 | to 100 % | to 100 % | to 100 % | to 100 % | to 100 % | to 100 % | to 100 % | to 100 % | to 100 % | to 100 % |

### Example 2: Viscosity properties of Compositions 1 to 9 in accordance with the invention and Comparative Composition A

The viscosities of the compositions given in Table 1 were measured using a Brookfield V2 viscometer (spindle RTV5, 1 minute, 20rpm) at 30°C and are shown in Table 2.

**Table 2: Viscosities of Compositions 1 to 9 in accordance with the invention and Comparative Composition A.**

| | **% active** | **A** | **1*** | **2*** | **3*** | **4*** | **5** | **6** | **7** | **8** | **9** |
|---|---|---|---|---|---|---|---|---|---|---|---|
| Viscosity, cP | | 300 | 7000 | 6000 | 24000 | 5000 | 23906 | 5628 | 5975 | 5801 | 7972 |

| | | | | | | | | | | | |
|---|---|---|---|---|---|---|---|---|---|---|---|
| *Predicted values | | | | | | | | | | | |

It will be seen that the comparative composition A, containing an AOS-CAPB combination, is too thin and shows minimal salt thickening response. In contrast, compositions in accordance with the invention show positive salt thickening over a range of surf ratios in the presence of cationic polymer.

## Claims

1. A sulphate free cleansing composition for hair, scalp and skin comprising, in an aqueous continuous phase:
a total amount of anionic surfactant, amphoteric surfactant and zwitterionic surfactant consisting of:
(i) from 3 wt % to 13 wt %, by weight of the total composition at 100 % activity, of an alpha olefin sulfonate anionic surfactant of general formula (I):
R¹-CH=CH-CH₂-SO₃⁻M⁺ (I)
in which R¹ is selected from linear or branched alkyl groups having from 11 to 13 carbon atoms and mixtures thereof; and M is a solubilizing cation;
(ii) from 1 to 6%, by weight of an amphoteric or zwitterionic surfactant, selected from an alkyl betaine of general formula (II)
R²-N⁺(CH₃)₂-CH₂-COO⁻M⁺ (II)
wherein R² = C12 (Lauryl) or Coco derived;
an alkyl hydroxy sultaine of general formula (III),
R³-N⁺(CH₃)₂-CH₂-CH(OH)-CH₂-SO₃⁻M⁺ (III)
wherein R³ = C12 (Lauryl) or Coco derived;
an alkyl aminopropyl hydroxy sultaine of general formula (IV),
R⁴-CO-NH-(CH₂)₃-N⁺(CH₃)₂-CH₂-CH(OH)-CH₂-SO₃⁻M⁺ (IV)
wherein R⁴ = C12 (Lauryl) or Coco derived;
an alkyl amphoacetate of general formula (V),
R⁵-CO-NH-(CH₂)₂-N(CH₂-CH₂-OH)(CH₂-COO⁻ M⁺) (V)
wherein R⁵ = C12 (Lauryl) or Coco derived;
and mixtures thereof; wherein no further anionic, amphoteric and zwitterionic surfactants are present;
(iii) from 0.05 wt % to 0.5 wt % of a cationic polymer;
(iv) an inorganic electrolyte; and
(v) water;
in which the weight ratio of (i) to (ii) ranges from 1:1 to 6:1 and the pH of the composition is from 3 to 6.5; and
wherein the composition has a viscosity from 3,500 to 15,000 mPa.s, when measured using a Brookfield V2 viscometer (spindle RTV5, 1 minute, 20rpm) at 30°C;
wherein the composition is free from thickening agents selected from thickening polymers and secondary surfactants not defined in (ii); and
wherein no other surfactants are present.

2. A composition according to claim 1, wherein the amount of alpha olefin sulfonate anionic surfactant of general formula (I) is from 3 to 12.85 %, preferably from 3.5 to 12 % by weight based on the total weight of the composition and at 100 % activity.

3. A composition according to claim 1 or claim 2, wherein the amount of amphoteric or zwitterionic surfactants of general formula (II), (III), (IV) or (V) is from 1 to 4 % (based on the total weight of the composition and 100 % activity).

4. A composition according to any preceding claim, in which the combined amount of (i) and (ii) ranges from 5 wt % to 15 wt % (by weight based on the total weight of the composition).

5. A composition according to any preceding claim, wherein the weight ratio of the alpha olefin sulfonate anionic surfactant (i) to the amphoteric or zwitterionic surfactant (ii) is from 1.5:1 to 4.5:1.

6. A composition according to any preceding claim, wherein the amphoteric or zwitterionic surfactant (ii) is selected from a betaine amphoteric surfactant of general formula (II), which is coco betaine, an amphoteric surfactant of general formula (III), which is lauryl hydroxy sultaine, and mixtures thereof, in an amount of from 1 to 4 % (by weight based on the total weight of the composition and 100 % activity).

7. A composition according to any preceding claim, wherein the cationic conditioning polymer is selected from Polyquaternium 10, guar hydroxypropyltrimethylammonium chlorides having a M_{w} ranging from 800,000 to 2.5 million g/mol and a charge density ranging from 0.5 to 1.8 meq/g, and mixtures thereof.

8. A composition according to any preceding claim, wherein the inorganic electrolyte is selected from the group consisting of metal chlorides, metal sulfates and mixtures thereof.

9. A composition according to any preceding claim, which has a viscosity from 4,000 to 12,000 mPa.s when measured using a Brookfield V2 viscometer (spindle RTV5, 1 minute, 20rpm) at 30°C.

10. A composition according to any preceding claim, which is transparent that is free from silicone.

11. A composition according to any preceding claim which comprises an isotropic surfactant phase.

12. A composition according to any preceding claim, which comprises an amount of inorganic electrolyte of from 1 to 3 wt % based on total weight of the composition, and wherein the weight ratio of (i) to (ii) is from 2:1 to 4:1.

13. A composition according to any preceding claim, which comprises an amount of inorganic electrolyte of from greater than 1 to 5 wt %, based on total weight of the composition, and wherein the weight ratio of (i) to (ii) is from from greater than 4:1 to 6:1, preferably 5:1 to 6:1.

14. A method of treating hair,scalp or skin comprising the step of applying to the hair, scalp or skin a composition as defined by any one of claims 1 to 13.

## Patentansprüche

1. Sulfatfreie Reinigungsmittelzusammensetzung für das Haar, die Kopfhaut und die Haut, umfassend in einer wässrigen kontinuierlichen Phase:
eine Gesamtmenge an anionischem Tensid, amphoterem Tensid und zwitterionischem Tensid, bestehend aus:
(i) 3 Gew.-% bis 13 Gew.-%, bezogen auf das Gewicht der Gesamtzusammensetzung bei 100% Aktivität, eines anionischen Alpha-Olefinsulfonat-Tensids der allgemeinen Formel (I):
R¹-CH=CH-CH₂-SO₃⁻M⁺ (I),
in welcher R¹ unter linearen oder verzweigen Alkylgruppen mit 11 bis 13 Kohlenstoffatomen und Mischungen davon ausgewählt ist und
M ein solubilisierendes Kation ist;
(ii) 1 bis 6 Gewichts-% eines amphoteren oder zwitterionischen Tensids, ausgewählt unter einem Alkylbetain der allgemeinen Formel (II)
R²-N⁺(CH₃)₂-CH₂-COO⁻M⁺ (II),
worin R² = C12 (Lauryl)- oder Coco-abgeleitet ist;
einem Alkylhydroxysultain der allgemeinen Formel (III),
R³-N⁺(CH₃)₂-CH₂-CH(OH)-CH₂-SO₃⁻M⁺ (III),
worin R³ = C12 (Lauryl)- oder Coco-abgeleitet ist;
einem Alkylaminopropylhydroxysultain der allgemeinen Formel (IV),
R⁴-CO-NH-(CH₂)₃-N⁺(CH₃)₂-CH₂-CH(OH)-CH₂-SO₃⁻M⁺ (IV),
worin R⁴ = C12 (Lauryl)- oder Coco-abgeleitet ist;
einem Alkylamphoacetat der allgemeinen Formel (V),
R⁵-CO-NH-(CH₂)₂-N(CH₂-CH₂-OH)(CH₂-COO⁻M⁺) (V),
worin R⁵ = C12 (Lauryl)- oder Coco-abgeleitet ist;
und Mischungen davon, wobei keine weiteren anionischen, amphoteren und zwitterionischen Tenside vorliegen;
(iii) 0,05 Gew.-% bis 0,5 Gew.-% eines kationischen Polymers;
(iv) einem anorganischen Elektrolyten; und
(v) Wasser;
wobei das Gewichtsverhältnis von (i) zu (ii) in dem Bereich von 1:1 bis 6:1 liegt und wobei der pH-Wert der Zusammensetzung 3 bis 6,5 beträgt; und
wobei die Zusammensetzung eine Viskosität von 3.500 bis 15.000 mPa.s aufweist, wenn unter Verwendung eines Brookfield V2-Viskosimeters (Spindel RTV5, 1 Minute, 20 U/min) bei 30°C gemessen;
wobei die Zusammensetzung frei von Verdickungsmitteln ist, ausgewählt unter Verdickungspolymeren und sekundären Tensiden, nicht in (ii) definiert; und
wobei keine weiteren Tenside vorliegen.

2. Zusammensetzung nach Anspruch 1, wobei die Menge des anionischen Alpha-Olefinsulfonat-Tensids der allgemeinen Formel (I) 3 bis 12,85 Gewichts-%, bevorzugt 3,5 bis 12 Gewichts-%, bezogen auf das Gesamtgewicht der Zusammensetzung und bei 100% Aktivität, beträgt.

3. Zusammensetzung nach Anspruch 1 oder Anspruch 2, wobei die Menge der amphoteren oder zwitterionischen Tenside der allgemeinen Formel (II), (III), (IV) oder (V) 1 bis 4% (bezogen auf das Gesamtgewicht der Zusammensetzung und 100% Aktivität) beträgt.

4. Zusammensetzung nach einem vorhergehenden Anspruch, worin die kombinierte Menge von (i) und (ii) in dem Bereich von 5 Gew.-% bis 15 Gew.-% (bezogen auf das Gesamtgewicht der Zusammensetzung) liegt.

5. Zusammensetzung nach einem vorhergehenden Anspruch, wobei das Gewichtsverhältnis des anionischen Alpha-Olefinsulfonat-Tensids (i) zu dem amphoteren oder zwitterionischen Tensid (ii) 1,5:1 bis 4,5:1 beträgt.

6. Zusammensetzung nach einem vorhergehenden Anspruch, wobei das amphotere oder zwitterionische Tensid (ii) ausgewählt ist unter einem amphoteren Betain-Tensid der allgemeinen Formel (II), welches Coco-Betain ist, einem amphoteren Tensid der allgemeinen Formel (III), welches Laurylhydroxysultain ist, und Mischungen davon, in einer Menge von 1 bis 4 Gewichts-% (bezogen auf das Gesamtgewicht der Zusammensetzung und 100% Aktivität).

7. Zusammensetzung nach einem vorhergehenden Anspruch, wobei das kationische Konditionierungspolymer unter Polyquaternium 10, Guarhydroxypropyltrimethylammoniumchloriden mit einem M_{w} in dem Bereich von 800.000 bis 2,5 Millionen g/mol und einer Ladungsdichte in dem Bereich von 0,5 bis 1,8 meq/g und Mischungen davon ausgewählt ist.

8. Zusammensetzung nach einem vorhergehenden Anspruch, wobei der anorganische Elektrolyt aus der Gruppe, bestehend aus Metallchloriden, Metallsulfaten und Mischungen davon, ausgewählt ist.

9. Zusammensetzung nach einem vorhergehenden Anspruch, die eine Viskosität von 4.000 bis 12.000 mPa.s aufweist, wenn unter Verwendung eines Brookfield V2-Viskosimeters (Spindel RTV5, 1 Minute, 20 U/min) bei 30°C gemessen wird.

10. Zusammensetzung nach einem vorhergehenden Anspruch, die transparent und frei von Silikon ist.

11. Zusammensetzung nach einem vorhergehenden Anspruch, die eine isotrope Tensidphase umfasst.

12. Zusammensetzung nach einem vorhergehenden Anspruch, die eine Menge an anorganischem Elektrolyt von 1 bis 3 Gew.-%, bezogen auf das Gesamtgewicht der Zusammensetzung, umfasst und wobei das Gewichtsverhältnis von (i) zu (ii) 2:1 bis 4:1 beträgt.

13. Zusammensetzung nach einem vorhergehenden Anspruch, die eine Menge an anorganischem Elektrolyten von mehr als 1 bis 5 Gew.-%, bezogen auf das Gesamtgewicht der Zusammensetzung, umfasst und wobei das Gewichtsverhältnis von (i) zu (ii) größer als 4:1 bis 6:1, bevorzugt 5:1 bis 6:1, beträgt.

14. Verfahren zur Behandlung von Haar, Kopfhaut oder Haut, umfassend den Schritt des Auftragens einer Zusammensetzung, wie in irgendeinem der Ansprüche 1 bis 13 definiert, auf das Haar, die Kopfhaut oder die Haut.

## Revendications

1. Composition nettoyante sans sulfate pour les cheveux, le cuir chevelu et la peau comprenant, dans une phase aqueuse continue :
une quantité totale de tensioactif anionique, tensioactif amphotère et tensioactif zwittérionique consistant en :
(i) 3 % en poids à 13 % en poids, par rapport au poids de la composition totale pour une activité de 100 %, d'un tensioactif anionique alpha-oléfinesulfonate de formule générale (I) :
R¹-CH=CH-CH₂-SO₃⁻M⁺ (I)
dans laquelle R¹ est choisi parmi les groupes alkyle linéaires ou ramifiés ayant 11 à 13 atomes de carbone et leurs mélanges ; et M est un cation solubilisant ;
(ii) 1 à 6 % en poids d'un tensioactif amphotère ou zwittérionique choisi parmi une alkylbétaïne de formule générale (II)
R²-N⁺(CH₃)₂-CH₂-COO⁻M⁺ (II)
dans laquelle R¹² est en C₁₂ (lauryle) ou est dérivé de coco ;
une alkylhydroxysultaïne de formule générale (III),
R³-N⁺(CH₃)₂-CH₂-CH(OH)-CH₂-SO₃⁻M⁺ (III)
dans laquelle R³ est en C₁₂ (lauryle) ou est dérivé de coco ;
une alkylaminopropylhydroxysultaïne de formule générale (IV),
R⁴-CO-NH-(CH₂)₃-N⁺(CH₃)₂-CH₂-CH(OH)-CH₂-SO₃⁻M⁺ (IV)
dans laquelle R⁴ est en C₁₂ (lauryle) ou est dérivé de coco ;
un amphoacétate d'alkyle de formule générale (V)
R⁵-CO-NH-(CH₂)₂-N(CH₂-CH₂-OH)(CH₂-COO⁻M⁺) (V)
dans laquelle R⁵ est en C₁₂ (lauryle) ou est dérivé de coco ;
et leurs mélanges ; dans laquelle aucun autre tensioactif anionique, amphotère ou zwittérionique n'est présent ;
(iii) 0,05 % en poids à 0,5 % en poids d'un polymère cationique ;
(iv) un électrolyte inorganique ; et
(v) de l'eau ;
dans laquelle le rapport en poids de (i) à (iii) est situé dans la plage allant de 1/1 à 6/1 et le pH de la composition est de 3 à 6,5 ; et
laquelle composition a une viscosité de 3 500 à 15 000 mPa.s quand elle est mesurée au moyen d'un viscosimètre Brookfield V2 (broche RTV5, 1 minute, 20 t/min) à 30°C ;
laquelle composition est exempte d'agents épaississants choisis parmi les polymères épaississants et les tensioactifs secondaires non définis en (ii) ; et
dans laquelle aucun autre tensioactif n'est présent.

2. Composition selon la revendication 1, dans laquelle la quantité de tensioactif anionique alpha-oléfinesulfonate de formule générale (I) est de 3 à 12,85 %, de préférence de 3,5 à 12 % en poids par rapport au poids total de la composition et pour une activité de 100 %.

3. Composition selon la revendication 1 ou la revendication 2, dans laquelle la quantité de tensioactifs amphotères ou zwittérioniques de formule générale (II), (III), (IV) ou (V) est de 1 à 4 % (par rapport au poids total de la composition et pour une activité de 100 %).

4. Composition selon l'une quelconque des revendications précédentes, dans laquelle la quantité combinée de (i) et (ii) est située dans la plage allant de 5 % en poids à 15 % en poids (par rapport au poids total de la composition).

5. Composition selon l'une quelconque des revendications précédentes, dans laquelle le rapport en poids du tensioactif anionique alpha-oléfinesulfonate (i) au tensioactif amphotère ou zwittérionique (ii) est de 1,5/1 à 4,5/1.

6. Composition selon l'une quelconque des revendications précédentes, dans laquelle le tensioactif amphotère ou zwittérionique (ii) est choisi parmi un tensioactif amphotère bétaïne de formule générale (II), qui est la (coprah-yl)bétaïne, un tensioactif amphotère de formule générale (III), qui est la laurylhydroxysultaïne, et leurs mélanges, en une quantité de 1 à 4 % (en poids par rapport au poids total de la composition et pour une activité de 100 %).

7. Composition selon l'une quelconque des revendications précédentes, dans laquelle le polymère conditionnant cationique est choisi parmi le Polyquaternium 10, les chlorures d'hydroxypropyltriméthylammonium-guar ayant une M_{w} située dans la plage allant de 800 000 à 2,5 millions de g/mol et une densité de charge située dans la plage allant de 0,5 à 1,8 méq/g, et leurs mélanges.

8. Composition selon l'une quelconque des revendications précédentes, dans laquelle l'électrolyte inorganique est choisi dans l'ensemble constitué par les chlorures métalliques, les sulfates métalliques et leurs mélanges.

9. Composition selon l'une quelconque des revendications précédentes, qui a une viscosité de 4 000 à 12 000 mPa.s quand elle est mesurée au moyen d'un viscosimètre Brookfield V2 (broche RTV5, 1 minute, 20 t/min) à 30°C.

10. Composition selon l'une quelconque des revendications précédentes, qui est transparente et qui est exempte de silicone.

11. Composition selon l'une quelconque des revendications précédentes, qui comprend une phase de tensioactif isotrope.

12. Composition selon l'une quelconque des revendications précédentes, qui comprend une quantité d'électrolyte inorganique de 1 à 3 % en poids par rapport au poids total de la composition, et dans laquelle le rapport en poids de (i) à (ii) est de 2/1 à 4/1.

13. Composition selon l'une quelconque des revendications précédentes, qui comprend une quantité d'électrolyte inorganique de plus de 1 à 5 % en poids par rapport au poids total de la composition, et dans laquelle le rapport en poids de (i) à (ii) est de plus de 4/1 à 6/1, de préférence de 5/1 à 6/1.

14. Méthode de traitement des cheveux, du cuir chevelu ou de la peau, comprenant l'étape d'application sur les cheveux, le cuir chevelu ou la peau d'une composition telle que définie par l'une quelconque des revendications 1 à 13.
